# EUROPÄISCHE PATENTSCHRIFT

(11) **EP 2 152 215 B1**
(45) Veröffentlichungstag und Bekanntmachung des Hinweises auf die Patenterteilung: **19.01.2011**
(21) Anmeldenummer: 08784504.6
(22) Anmeldetag: 30.05.2008
(51) Int. Cl.: A61F 13/20

(54) **TAMPON SOWIE VERFAHREN ZUR HERSTELLUNG EINES TAMPONS**
TAMPON, AND METHOD FOR THE PRODUCTION OF A TAMPON
TAMPON ET SON PROCÉDÉ DE PRODUCTION

(30) Priorität: 01.06.2007 DE 102007025783
(43) Veröffentlichungstag der Anmeldung: 17.02.2010
(73) Patentinhaber: Ruggli Projects AG, 6332 Hagendorn (CH)
(72) Erfinder: MÜLLER, Peter, CH-8004 Zürich (CH); ROLLI, Kilian, CH-5303 Würenlingen (CH)
(74) Vertreter: Ofner, Clemens
(86) Internationale Anmeldenummer: PCT/EP2008/004289
(87) Internationale Veröffentlichungsnummer: WO 2008/145370

(56) Entgegenhaltungen:
- EP-A- 0 149 155
- EP-A- 1 108 407
- DE-A1- 10 316 234
- DE-C- 818 234
- US-A1- 2007 016 156

## Beschreibung

Die vorliegende Erfindung betrifft einen Tampon mit einem Einführende und einem rückwärtigen Ende, bestehend aus einem verpressten saugfähigen Streifen und einem aus dem rückwärtigen Ende herausgeführten Rückholfaden. Ferner betrifft die Erfindung ein Verfahren zur Herstellung eines Tampons aus einem saugfähigen, mit einem Rückholfaden verbundenen Streifen.

Bei heutigen sogenannten Applikator-Tampons, bei denen sich also der eigentliche Tampon innerhalb einer Hülse befindet und durch Verschieben eines Stempels appliziert wird, ist der für die spätere Entfernung des benutzten Tampons erforderliche Rückholfaden durch Nähen an dem saugfähigen Material des Tampons befestigt. Bei der Herstellung des Tampons dient ein saugfähiger Streifen als Ausgangsmaterial, auf welchen der Rückholfaden aufgenäht wird. In einem anschließenden Schritt erfolgt dann unter Druckeinwirkung die Formgebung des Tampons von der zunächst streifenförmigen Form in die etwa zylindrische Endform.

Das Vernähen des Rückholfadens mit dem saugfähigen Streifen des Tampons hat sich als unvorteilhaft erwiesen. Denn den Nähmaschinen sind hinsichtlich der erreichbaren Nähgeschwindigkeiten Grenzen gesetzt, weshalb es nicht möglich ist, den Produktionsablauf einer Tampon-Fertigung über die Geschwindigkeit der Nähmaschine hinaus zu erhöhen, und so kurze Fertigungszeiten zu erreichen.

Es ist daher die Aufgabe der vorliegenden Erfindung, einen Tampon sowie ein Verfahren zu dessen Herstellung bereitzustellen, die sich durch kurze Fertigungszeiten auszeichnen.

Zur Lösung dieser Aufgabe wird in Bezug auf den Tampon vorgeschlagen, dass der Rückholfaden in Gestalt einer Schlinge um den Streifen herumgeführt ist, und dass der Streifen zu jeder Seite der Schlinge mit einer Knickung versehen ist.

Durch das Festlegen des Rückholfadens an dem saugfähigen Streifen unter Bildung einer um den Streifen herumgeführten Schlinge lassen sich bei der Produktion des Applikatortampons hohe Fertigungsgeschwindigkeiten bzw. Taktzeiten erreichen. Die Schlinge bewirkt eine vorläufige Sicherung des Rückholfadens. Während des anschließenden Pressens des-Applikatortampons kommt es zum Entstehen von Knicken im Material des Streifens, welche Knicke die Beweglichkeit des Rückholfadens begrenzen mit der Folge, dass es in dieser Phase zu keinem Verrutschen oder einem Verlust des Fadens mehr kommen kann.

Zur zusätzlichen Sicherung des Rückholfadens kommen Verbindungsverfahren, z.B. Schweißverfahren in Betracht. Vor allem das Verschweißen mittels beheizten Druckstücken, durch Ultraschall, mittels Laser usw., ist hierzu geeignet. Aber auch ein Klebeprozess ist geeignet, das Rückholband noch besser mit dem anschließend verformten Streifen zu verbinden.

Vorteilhaft ist der saugfähige Streifen aus einem Flüssigkeit speichernden Faser-, Vlies- oder Watteband und einer dieses umgebenden Umhüllung zusammengesetzt. Das im Inneren des Streifens vorgesehene Faserband speichert die durch die Umhüllung hindurch tretende Flüssigkeit.

Für die Zugfestigkeit der Verbindung des Rückholfadens mit dem Streifen ist es ferner von Vorteil, wenn der Rückholfaden sowohl entlang der Oberseite des Streifens wie auch dessen Unterseite verschweißt bzw. verklebt ist.

In verfahrensmäßiger Hinsicht wird zur Lösung der vorstehenden Aufgabe ein Verfahren zur Herstellung eines Tampons aus einem saugfähigen, mit einem Rückholfaden verbundenen Streifen mit den folgenden Schritten vorgeschlagen:
a) Zuschneiden des saugfähigen Streifens,
b) Platzierung eines Rückholfadens in Gestalt einer um den Streifen herumgelegten Schlinge,
c) Pressen des Streifens zwischen aufeinander zu beweglichen Backen unter Bildung einer Knickung des Streifens zu jeder Seite der Schlinge.

Mit diesem Verfahren lässt sich eine gegenüber dem Stand der Technik deutlich höhere Fertigungsgeschwindigkeit, d.h. eine kürzere Fertigungszeit pro Tampon, erreichen.

Weitere Einzelheiten und Vorteile eines erfindungsgemäßen Tampons sowie eines erfindungsgemäßen Verfahrens zu dessen Herstellung werden nachfolgend unter Zuhilfenahme der beigefügten Zeichnungen erläutert. Darin zeigen:
- Fig. 1: eine perspektivische Darstellung der Herstellung eines Endlosmaterials, aus welchem gemäß
- Fig. 2: einzelne saugfähige Streifen abgeschnitten werden,
- Fig. 3: vier verschiedene Streifengeometrien (Fign. 3a bis 3d),
- Fig. 4: eine perspektivische Anordnung zum Verschweißen des Rückholfadens mit dem Streifen,
- Fig. 5: die Formgebung des Applikatortampons durch aufeinander zu gerichtete Druckeinwirkung in einzelnen Stufen Fign. 5a, 5b und 5c.

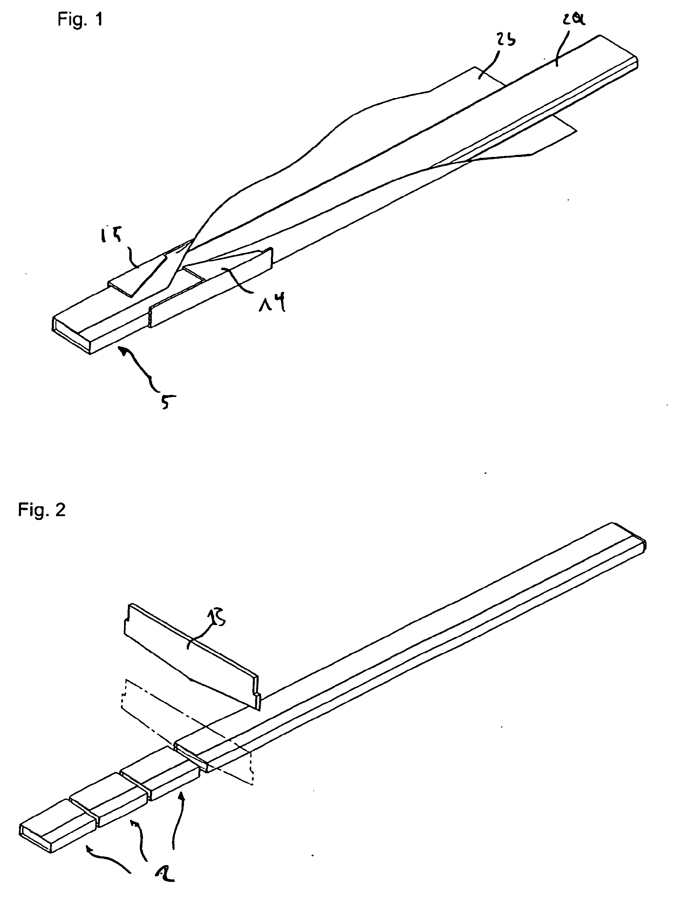

Dargestellt ist in Fig. 1 zunächst die Herstellung eines Endlosmaterials 5, von welchem in sich anschließenden Fertigungsschritten saugfähige Streifen 2 zur Herstellung eines Tampons 1 zum Einsatz in einem Tampon-Applikator getrennt werden.
Das Endlosmaterial 5 besteht aus einem für Flüssigkeiten saugfähigen Faserband oder Watteband 2a sowie einer das Band umschließenden Umhüllung 2b. Das Watteband 2a weist einen in etwa eckigen oder ovalen Querschnitt auf, wohingegen die Umhüllung 2b eine vergleichsweise dünne, für Flüssigkeit passierbare Folie oder
Membran ist. Die Einhüllung des Faser- oder Wattebandes 2a erfolgt durch eine Relativbewegung des Wattebandes 2a sowie der Folie 2b gegenüber zwei Anlagebacken 14, 15, wodurch die Umhüllung 2b ähnlich dem Stanniolpapier eines Käugummistreifens um das Watteband 2a herumgelegt wird.

Das so vorbereitete Endlosmaterial 5 wird im nächsten Fertigungsschritt einer oder auch mehreren Schneiden 13 zugeführt, die saugfähige Streifen 2 von dem Endlosmaterial 5 abtrennen. Als Schneiden 13 sind eine Vielzahl verschiedener Schneiden sowie auch verschiedener Anordnungen von Schneiden denkbar.

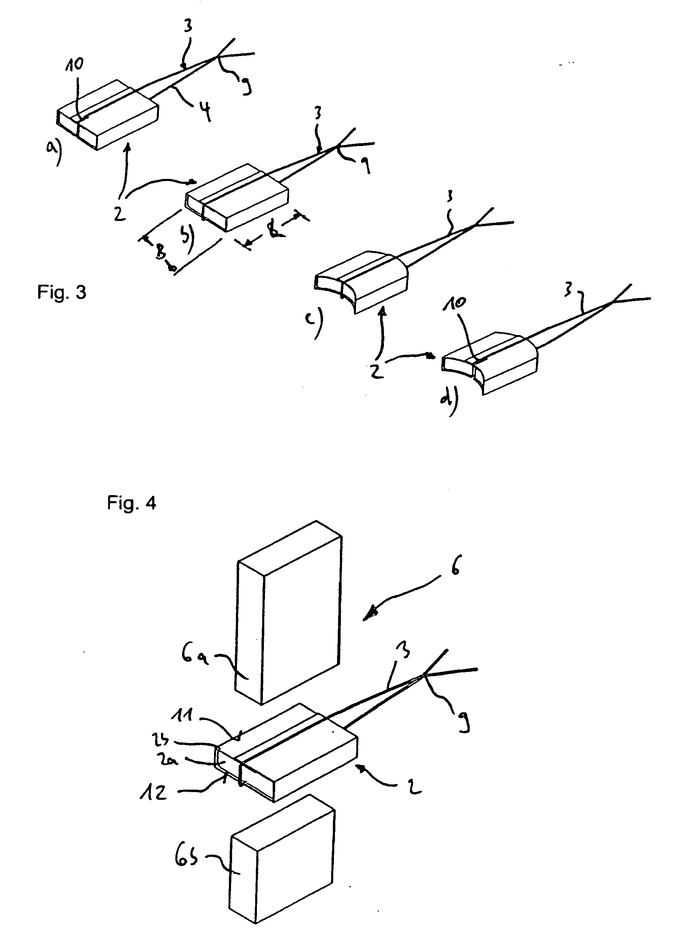

Die Geometrie einiger saugfähiger Streifen 2 ist in den Fign. 3a bis 3d dargestellt. Die in den Fign. 3a und 3b abgebildeten Streifen 2 sind in etwa kubisch, wobei der in Fig. 3a dargestellte Streifen an einer Seite mit einem in Längsrichtung des Streifens verlaufenden, kurzen Schlitz 10 zum Einklemmen eines zu einer Schlinge 4 geformten Rückholfadens 3 versehen ist. Eingeklemmt ist die Schlinge 4 im Bereich ihres Umkehrpunktes. Auch der in Fig. 3d dargestellte Streifen 2 ist mit einem solchen Schlitz oder auch mit einer Kerbe 10 versehen. Abweichend zu den in den Fign. 3a und 3b dargestellten Streifen 2 ist der Streifen 2 aus den Fign. 3c und 3d nicht von kubisch-rechteckiger, sondern von in etwa schaufelförmiger Gestalt.

Sämtliche Streifen 2 weisen eine Länge L sowie eine Breite B auf. Ferner lässt Fig. 3 erkennen, dass das ungefähr auf der Mitte der Breite B zu einer Schlinge 4 gelegte Rückholband 3 an seinem hinteren, freien Ende mit einem Knoten 9 versehen ist. Auf diese Weise entsteht eine geschlossene Schlaufe, die den saugfähigen Streifen 2 in Längsrichtung einmal umschlingt, d.h. der saugfähige Streifen 2 befindet sich innerhalb der Schlaufe. Nachdem der saugfähige Streifen 2 und das Rückholband 3 derart vorbereitet und zueinander platziert sind, können sie im nächsten Fertigungsschritt zusätzlich miteinander verbunden werden.

In Fig. 4 ist zu diesem Zweck eine Anordnung zum Verschweißen des Rückholfadens 3 mit dem saugfähigen Streifen 2 dargestellt. Beim Ausführungsbeispiel erfolgt das Schweißen über jeweils ein oberhalb bzw. unterhalb des Streifens 2 angeordnetes, beheiztes Druckstück 6a, 6b einer Schweißeinrichtung 6. Über die einander zugewandten Druckflächen der Druckstücke 6a, 6b wird das Rückholband 3 unter Temperatureinwirkung mit einer definierten Kraft beaufschlagt, bis eine Schweißverbindung zwischen dem saugfähigen Streifen 2 und dem Rückholband 3 entsteht. Je nach Wahl der Schweißparameter, wie etwa Druck, Temperatur, Schweißzeit usw., kann das Rückholband 3 nur mit dem Non-Woven-Material der Umhüllung 2b verschweißt werden, oder auch zusätzlich mit dem Watteband 2a. Wie Fig. 4 weiter erkennen lässt, erfolgt die Verschweißung des Rückholbandes 3 entlang der Oberseite 11 sowie der Unterseite 12 des saugfähigen Streifens 2. Denkbar sind aber auch Anordnungen, bei denen z.B. nur ein Faden mit entweder der Ober- oder der Unterseite verschweißt ist. Anstelle des Verschweißens ist auch z.B. ein Verkleben möglich.

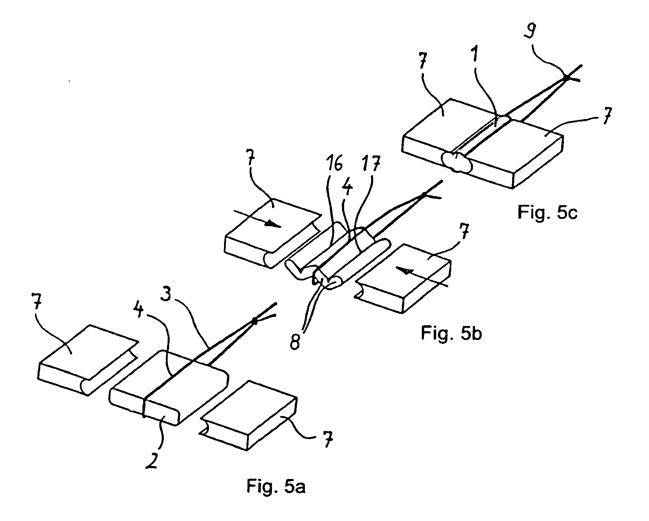

In den Fign. 5a bis 5c ist in drei Stufen die Überführung des saugfähigen Streifens 2 von seiner kubischen bzw. schaufelförmigen Gestalt in eine im Wesentlichen zylindrische Form dargestellt. In einem ersten Schritt (Fig. 5a) wird der saugfähige Streifen 2 mit dem mittig darauf angeordneten und ggfs. durch Verschweißen fixierten Rückholband 3 zwischen zwei radial aufeinander zu bewegliche Pressbacken 7 einer Presse positioniert. Durch einfaches oder mehrstufiges Pressen wird der saugfähige Streifen 2 dann gemäß den Fign. 5b und 5c in eine in etwa zylindrische Form gedrückt, wobei sich der ursprünglich flache Streifen 2 unter Bildung von mehreren Knickungen 16, 17 in mehrere Schichten 8 faltet. Das Knicken bzw. Falten erfolgt in Zick-Zack-Form, etwa in der Querschnittsgestalt eines "W'. Die Kantenlänge L (Fig. 3b) des Streifens 2 bleibt dabei erhalten, d.h. sie entspricht in etwa der Länge des fertigen Tampons zwischen seinem Einführende und seinem rückwärtigen Ende.

Die sich bereits zu Beginn der Verformung ausbildenden Knicke 16, 17 führen zu einer Begrenzung, zumindest aber zu einer Behinderung der seitlichen Beweglichkeit des Rückholfadens 3 mit der Folge, dass es während des weiteren Verpressens des Streifens 2 zur endgültigen Tamponform zu keinem Verrutschen oder einem Verlust des Fadens kommen kann. Dieser Vorteil wird ungeachtet der bereits beschriebenen Möglichkeit erzielt, den Rückholfaden in einem vorangehenden Fertigungsschritt zu verschweißen oder zu verkleben. Ferner ist es möglich, die Umhüllung 2b erst zu einem Zeitpunkt um den Materialstreifen 2 zu legen, zu dem der Rückholfaden 3 bereits in Form der Schlaufe um den jeweiligen Streifen 2 gelegt worden ist.

Nach erfolgter Formgebung, d. h. dem Verpressen, wird der Tampon dann in die Hülse des Tampon-Applikators hineingesetzt. Derartige, zumeist aus zwei teleskopierbaren Kunststoffhülsen zusammengesetzte Applikatoren sind bekannt.

Mit dem Tampon bzw. dem Verfahren zu dessen Herstellung lassen sich gegenüber dem Stand der Technik verbesserte Fertigungszeiten erreichen. So ist es z.B. bei bekannten Maschinen zur Herstellung von Applikator-Tampons derzeit kaum möglich, pro zugeführter Endlosmaterialbahn mehr als 70 Tampons pro Minute zu produzieren. Für höhere Tamponzahlen wäre es erforderlich, der Maschine mehrere Endlosmaterialbahnen zuzuführen. Durch das Umschlingen des Rückholfadens werden diese Nachteile überwunden, so dass pro Endlosbahn bis zu 120 Tampons pro Minute produziert und in die entsprechenden Applikatoren eingesetzt werden können.

### Bezugszeichenaufstellung

- 1: Tampon
- 2: saugfähiger Streifen
- 2a: Watteband
- 2b: Umhüllung
- 3: Rückholfaden
- 4: Schlinge, Schlaufe
- 5: Endlosmaterial
- 6: Schweißeinrichtung
- 6a: oberes Druckstück
- 6b: unteres Druckstück
- 7: radiale Pressbacke
- 8: Schicht
- 9: Knoten
- 10: Kerbe, Schlitz
- 11: Oberseite
- 12: Unterseite
- 13: Schneide
- 14: Anlagebacke
- 15: Anlagebacke
- 16: Knickung
- 17: Knickung

- B: Breite
- L: Länge

## Patentansprüche

1. Tampon mit einem Einführende und einem rückwärtigen Ende, bestehend aus einem verpressten saugfähigen Streifen (2) mit einer Breite (B) und einer Länge (L), die einer Kantenlänge (L) des Streifens (2) und in etwa der Länge des fertigen Tampons zwischen einem Einführende und einem rückwärtigem Ende entspricht und einem aus dem rückwärtigen Ende herausgeführten Rückholfaden (3), wobei der Rückholfaden (3) in Gestalt einer Schlinge den Streifen (2) ungefähr auf der Mitte der Breite (B) in Richtung der Länge (L) einmal umschlingt und an einem hinteren freien Ende mit einem Knoten (9) versehen ist, **dadurch gekennzeichnet, dass** der Streifen bei gleicher Länge (L) in Zick-Zack-Form in mehreren Schichten gefaltet ist, zwischen denen mehrere Knickungen (16, 17) gebildet sind und auf jeder Seite der Schlinge eine Knickung (16, 17) ausgebildet ist.

2. Tampon nach Anspruch 1, **dadurch gekennzeichnet, dass** der zick-zack-förmig unter Bildung der Knickungen (16, 17) verpresste Streifen (2) eine im Wesentlichen zylindrische Form aufweist.

3. Tampon nach Anspruch 1 oder 2, **dadurch gekennzeichnet, dass** der Rückholfaden (3) mit seiner Schlinge (4) im Bereich des Umkehrpunktes in einem an einer Seite in einen in Richtung der Länge (L) des Streifens (2) verlaufenden kurzen Schlitz (10) oder eine Kerbe eingeklemmt ist.

4. Tampon nach Anspruch 1 oder 2, **dadurch gekennzeichnet, dass** der saugfähige Streifen (2) aus einem zur Flüssigkeitsspeicherung geeigneten Faser- oder Watteband (2a) und einer dieses umgebenden Umhüllung (2b) zusammengesetzt ist.

5. Tampon nach einem der Ansprüche 1 bis 4, **dadurch gekennzeichnet, dass** der Rückholfaden (3) durch Verschweißen mit dem saugfähigen Streifen (2) oder der Umhüllung (2b) verbunden ist.

6. Tampon nach einem der Ansprüche 1 bis 4, **dadurch gekennzeichnet, dass** der Rückholfaden (3) mit dem saugfähigen Streifen (2) oder der Umhüllung (2b) verklebt ist.

7. Tampon nach Anspruch 5 oder 6, **dadurch gekennzeichnet, dass** der Rückholfaden (3) sowohl entlang der Oberseite (11) des Streifens (2) wie auch entlang dessen Unterseite (12) verschweißt bzw. verklebt ist.

8. Verfahren zur Herstellung eines Tampons aus einem saugfähigen, mit einem Rückholfaden (3) verbundenen Streifen (2), bestehend aus folgenden Schritten:
a) Abtrennung eines saugfähigen Streifens (2) mit einer Breite (B) und einer Länge (L), die einer Kantenlänge des Streifens (2) und in etwa der Länge des fertigen Tampons zwischen einem Einführende und einem rückwärtigen Ende entspricht;
b) Umschlingen des Streifens (2) ungefähr auf der Mitte der Breite (B) in Richtung der Länge (L) mit einem Rückholfaden (3);
c) Verknoten des Rückholfadens (3) an einem hinteren freien Ende;
d) Falten des Streifens (2) bei gleicher Länge (L) in Zick-Zack-Form zu mehreren Schichten durch Zusammenpressen von aufeinander zu beweglichen Backen (7) unter Bildung von Knickungen (16, 17) des Streifens (2) zu jeder Seite der Schlinge (4);
e) Einfaches oder mehrstufiges Pressen des Streifens (2) zu einer etwa zylindrischen Form.

9. Verfahren nach Anspruch 8, **dadurch gekennzeichnet, dass** der Streifen (2) mit einer Umhüllung (2b) versehen wird.

10. Verfahren nach Anspruch 8 oder 9, **dadurch gekennzeichnet, dass** der Rückholfaden (3) durch Verschweißen mit dem saugfähigen Streifen (2) oder der Umhüllung (2b) verbunden wird.

11. Verfahren nach Anspruch 8 oder 9, **dadurch gekennzeichnet, dass** der Rückholfaden (3) durch Verkleben mit dem saugfähigen Streifen (2) oder der Umhüllung (2b) verbunden wird.

## Claims

1. Tampon with an insertion end and a rear end, made from a pressed absorbent strip (2) with a width (B) and a length (L) which corresponds to an edge length (L) of the strip (2) and to approximately the length of the finished tampon between an insertion end and a rear end, and a withdrawal string (3) running out from the rear end, which withdrawal string (3) is placed in the shape of a loop approximately at the centre of the width (B) wrapped around the strip (2) once in the direction of the length (L) and is provided with a knot (9) at its rear free end, **characterised in that** the strip is folded, preserving the same length (L), in a zig-zag shape in several layers between which several folds (16, 17) are formed, and a fold (16, 17) is formed on each side of the loop.

2. Tampon as claimed in claim 1, **characterised in that** the strip (2) pressed in a zig-zag shape forming the folds (16, 17) is of an essentially cylindrical shape.

3. Tampon as claimed in claim 1 or 2, **characterised in that** the withdrawal string (3) is clamped by its loop (4) in the region of the point at which it is turned back on itself in a short slit (10) or a notch on a face extending in the direction of the length (L) of the strip (2).

4. Tampon as claimed in claim 1 or 2, **characterised in that** the absorbent strip (2) is made up of a fibre or wadding strip (2a) suitable for storing liquid and a sheath (2b) enclosing it.

5. Tampon as claimed in one of claims 1 to 4, **characterised in that** the withdrawal string (3) is joined to the absorbent strip (2) or to the sheath (2b) by welding.

6. Tampon as claimed in one of claims 1 to 4, **characterised in that** the withdrawal string (3) is bonded to the absorbent strip (2) or to the sheath (2b).

7. Tampon as claimed in claim 5 or 6, **characterised in that** the withdrawal string (3) is welded or bonded both along the top face (11) of the strip (2) and along its bottom face (12).

8. Method of producing a tampon from an absorbent strip (2) joined to a withdrawal string (3), comprising the following steps:
a) cutting an absorbent strip (2) with a width (B) and a length (L) which corresponds to an edge length of the strip (2) and approximately the length of the finished tampon between an insertion end and a rear end;
b) placing a withdrawal string (3) in a loop around the strip (2) more or less at the centre of the width (B) in the direction of the length (L);
c) knotting the withdrawal string (3) at a rear free end;
d) folding the strip (2), preserving the same length (L), in a zig-zag shape to form several layers by pressing together jaws (7) which can be moved towards one another, forming folds (16, 17) in the strip (2) on each side of the loop (4);
e) pressing the strip (2) in a single or multiple-stage pressing operation to an approximately cylindrical shape..

9. Method as claimed in claim 8, **characterised in that** the strip (2) is provided with a sheath (2b).

10. Method as claimed in claim 8 or 9, **characterised in that** the withdrawal string (3) is joined to the absorbent strip (2) or to the sheath (2b) by welding.

11. Method as claimed in claim 8 or 9, **characterised in that** the withdrawal string (3) is joined to the absorbent strip (2) or to the sheath (2b) by bonding.

## Revendications

1. Tampon avec une extrémité d'insertion et une extrémité arrière, constitué d'une bande absorbante compressée (2) avec une largeur (B) et une longueur (L) qui correspond à une longueur d'arête (L) de la bande (2) et à peu près à la longueur du tampon fini entre l'extrémité d'insertion et une extrémité arrière et d'un fil de récupération (3) sortant de l'extrémité arrière, où le fil de récupération (3), sous la forme d'une boucle, entoure une fois la bande (2) à peu près au milieu de la largeur (B) dans la direction de la longueur (L) et est pourvu à une extrémité arrière libre d'un noeud (9), **caractérisé en ce que** la bande, pour une même longueur (L), est pliée en une forme zigzag en plusieurs couches entre lesquelles sont formés plusieurs plis (16, 17), et de chaque côté de la boucle, un pli (16, 17) est réalisé.

2. Tampon selon la revendication 1, **caractérisé en ce que** la bande (2) compressée en zigzag en formant les plis (16, 17) présente une forme sensiblement cylindrique.

3. Tampon selon la revendication 1 ou 2, **caractérisé en ce que** le fil de récupération (3), avec sa boucle (4), est coincé dans la zone du point d'inversion dans une fente courte (10) ou une entaille s'étendant à un côté dans la direction de la longueur (L) de la bande (2).

4. Tampon selon la revendication 1 ou 2, **caractérisé en ce que** la bande d'absorption (2) est constituée d'une bande de fibres ou de coton (2a) apte à stocker du liquide et d'une enveloppe (2b) entourant celle-ci.

5. Tampon selon l'une des revendications 1 à 4, **caractérisé en ce que** le fil de récupération (3) est relié par soudage à la bande absorbante (2) ou à l'enveloppe (2b).

6. Tampon selon l'une des revendications 1 à 4, **caractérisé en ce que** le fil de récupération (3) est collé à la bande absorbante (2) ou à l'enveloppe (2b).

7. Tampon selon la revendication 5 ou 6, **caractérisé en ce que** le fil de récupération (3) est soudé respectivement collé à la fois le long du côté supérieur (11) de la bande (2) et aussi le long de son côté inférieur (12).

8. Procédé de fabrication d'un tampon en une bande absorbante (2) reliée à un fil de récupération (3), comprenant les étapes suivantes:
a) séparation d'une bande absorbante (2) avec une largeur (B) et une longueur (L) qui correspond à une longueur d'arête de la bande (2) avec une largeur (B) et une longueur (L) qui correspond à une longueur d'arête de la bande (2) et à peu près à la longueur du tampon fini entre une extrémité d'insertion et une extrémité arrière;
b) entourage de la bande (2) à peu près au milieu de la largeur (B) en direction de la longueur (L) avec un fil de récupération (3);
c) nouage du fil de récupération (3) à son extrémité arrière libre;
d) pliage de la bande (2) pour une même longueur (L) en forme de zigzag en plusieurs couches par compression de mâchoires (7) déplaçables l'une vers l'autre en formant des plis (16, 17) de la bande (2) de chaque côté de la boucle (4);
e) pressage simple ou multiple de la bande (2) en une forme à peu près cylindrique.

9. Procédé selon la revendication 8, **caractérisé en ce que** la bande (2) est pourvue d'une enveloppe (2b).

10. Procédé selon la revendication 8 ou 9, **caractérisé en ce que** le fil de récupération (3) est relié par soudage à la bande absorbante (2) ou à l'enveloppe (2b).

11. Procédé selon la revendication 8 ou 9, **caractérisé en ce que** le fil de récupération (3) est relié par collage à la bande absorbante (2) ou à l'enveloppe (2b).
